(19) Europäisches Patentamt
European Patent Office
Office européen des brevets

(11) **EP 4 190 294 A1**

(12) **EUROPEAN PATENT APPLICATION**

(43) Date of publication:
**07.06.2023 Bulletin 2023/23**

(21) Application number: **21212015.8**

(22) Date of filing: **02.12.2021**

(51) International Patent Classification (IPC):
**A61F 13/49** (2006.01)   **A61F 13/496** (2006.01)

(52) Cooperative Patent Classification (CPC):
**A61F 13/49003; A61F 13/49006;**
A61F 2013/530489

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fameccanica.Data S.p.A.**
**66020 San Giovanni Teatino (CH) (IT)**

(72) Inventor: **LUPINETTI, Serafino**
**I-66020 San Giovanni Teatino (Chieti) (IT)**

(74) Representative: **Marchitelli, Mauro**
**Buzzi, Notaro & Antonielli d'Oulx S.p.A.**
**Corso Vittorio Emanuele II, 6**
**10123 Torino (IT)**

(54) **A WASHABLE ABSORBENT GARMENT**

(57)    A washable absorbent garment comprising a back layer (12), a washable absorbent core (28) comprising a washable absorbent pad, and a top layer (30) which covers the washable absorbent core (28), wherein the washable absorbent comprises a blend of superabsorbent fibers, polyolefin fibers and polyester fibers, and has a performance ratio, defined as ratio between absorbent capacity (g/g) and thickness (mm), comprised between 1 and 15 g/g·mm, wherein the thickness is equal to or lower than 7 mm.

fig. 1

## Description

### Field of the invention

The present invention relates to absorbent garment for the absorption and retention of liquids, and more particularly to washable and reusable absorbent garments.

### Prior art

Various washable garments having a pant-like configuration and useful as incontinence garments, menstrual garments, training garments, diapers, and the like, are commercially available, as well as being disclosed in various patents.

For instance, absorbent garments are commercially available which are machine washable, and thus reusable, having an absorbent pad permanently connected to the pant structure. For instance, GB2176692A discloses an absorbent sanitary garment made in the form of knitted polyester material which incorporates a non-removable pad made of brushed polyester which has the appearance of soft felt. The absorbent pad cannot be removed and is made of a material which can be frequently washed.

Nowadays, washable absorbent garments have an absorbent pad made of natural raw materials, such as cotton or bamboo, whose absorbent power is relatively low, so that - to meet the absorbency requirements - the absorbent pad must be very thick (10-50 mm). The high thickness, which is obviously associated with an equally high weight, make the absorbent garment unsightly, uncomfortable to wear and therefore unattractive to end users.

There is therefore a need to provide washable garments with a thinner and lighter permanent absorbent pad without compromising absorbency.

### Object and summary of the invention

The object of the present invention is to provide a washable absorbent garment which overcomes the problems of the prior art.

According to the present invention, this object is achieved by a washable absorbent garment having the features of claim 1.

The claims form an integral part of the disclosure provided here in relation to the invention.

### Brief description of the drawings

The present invention will now be described in detail with reference to the attached drawings, given purely by way of non-limiting example, wherein:

- Figure 1 is a perspective view of a washable absorbent garment in an open configuration,
- Figure 2 is a plan view of the absorbent garment of figure 1 in the open configuration,
- Figure 3 is a schematic cross-section taken along the line III-III of figure 2.

It will be appreciated that the various figures may not be represented on the same scale. It will also be appreciated that in some figures certain elements or components may not be shown for a better understanding.

### Detailed description

The washable absorbent garment object of the present description is suitable to be worn for containing and absorbing discharges resulting from light urinary incontinence and/or menstruation. The washable absorbent garment object of the present description is in fact able to absorb up to 150 ml of urine and/or blood.

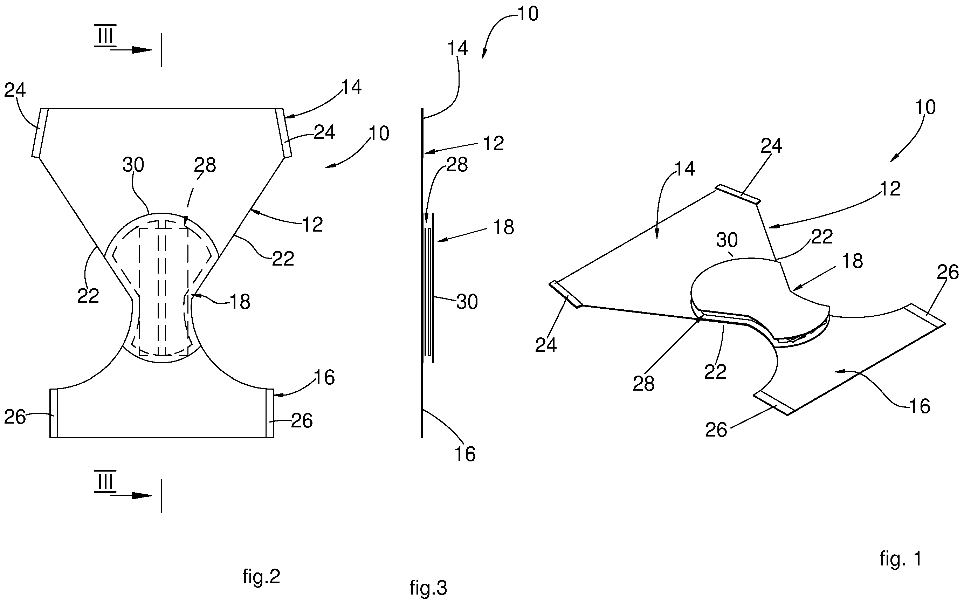

With reference to figures 1-3 the numeral reference 10 indicates a washable absorbent garment.

The washable absorbent garment 10 comprises a back layer 12 of washable fabric forming a pant structure. The back layer 12 has rear and front waist sections 14, 16 and a crotch section 18 intermediate between the rear and front waist sections 14, 16. The rear and front waist sections 14, 16 are wider than the crotch section 18, such that in the extended position of figures 1 and 2 the back layer 12 has substantially an hourglass shape.

The back layer 12 is a fabric formed by woven threads which is washable as ordinary textile garments, e.g. swimsuits, underwear, etc. The back layer 12 may be made of any natural or synthetic material used for producing textile articles, e.g. cotton, Lycra®, nylon, polyester, polypropylene, polyamide, elastane, Econyl® etc. and any mixture thereof. In possible embodiments, the back layer 12 may be an elastic fabric made of a mixture of polyamide and elastane, cotton and elastane e.g. 80% polyamide/cotton and 20% elastane. The back layer 20 may have a weight comprised between 100-200 g/m$^2$.

The crotch section 18 of the back layer 12 has two curved side edges 22 shaped to conform to the legs of the user in the configuration in which the washable absorbent garment 10 is worn. The rear and front waist sections 14, 16 of the back layer 12 have respective side edges 24, 26 which are joined to each other such that the washable absorbent garment 10 is closed in a pant-like shape and has two leg openings on opposite sides of the crotch section 18.

The side edges 24, 26 are joined to each other by welding or by glue. Welding may be ultrasonic welding, laser welding, or mechanical welding.

The washable absorbent garment 10 comprises a washable absorbent core 28 permanently fixed in the crotch section 18 of the back layer 12. The washable absorbent core 28 extends only on the crotch section 18 of the back layer 12 and does not extend on the rear and front waist sections 14, 16 of the back layer 12.

The washable absorbent garment 10 comprises a top layer 30 of washable fabric which covers the washable absorbent core 28. The top layer 30 may be made of the same raw materials as the fabric forming the back layer 12. The top layer 30 completely covers the washable absorbent core 28 but does not extend on the rear and front waist sections 14, 16 of the back layer 12.

The top layer 30 and the washable absorbent core 28 are permanently joined to the back layer 12 by welding or by glue. Welding may be ultrasonic welding, laser welding, or mechanical welding.

The washable absorbent core 28 includes a washable absorbent pad, formed by absorbent fibers and, optionally, decontaminating and/or functionalizing materials and/or absorbent materials, and two external closure sheets enclosing in a sandwich configuration the washable absorbent pad. The two external closure sheets have the function of containing the fibers (and the other materials, if present), and giving structure to the washable absorbent core 28, which without them would tend to lose and release the absorbent fibers during washing cycles, that over time would affect the proper functioning of the same.

The washable absorbent garment according to the present invention retains an absorbent capacity of more than 80% for up to 40 wash cycles.

The absorbent fibers comprising the washable absorbent pad are needlefelt, thermal bonded, laminated non-woven absorbent fibers.

The absorbent fibers comprising the washable absorbent pad are a blend of superabsorbent fibers, and polyolefin and/or polyester fibers.

The washable absorbent pad according to the present invention has a performance ratio (PR), defined as ratio between absorbent capacity (expressed as g/g) and thickness (expressed in mm), comprised between 1 and 15 g/g·mm wherein the thickness is equal to or lower than 7 mm. The absorbent capacity (AC) of the washable absorbent pad is defined as ratio between the amount of 0.9% saline solution or water absorbed per square meter (AB) and the grammage (GSM):

$$AC = AB \ (g/m^2) \ / \ GSM \ (g/m^2).$$

The performance ratio range comprised between 1 and 15 g/g·mm guarantees absorbency and fluid handling of the washable absorbent pad adequate to absorb up to about 100 ml of urine and/or blood.

The washable absorbent pad has an absorbent capacity comprised between 5 and 25 g/g and a thickness comprised between 1 and 7 mm.

The fibers blend comprising the washable absorbent pad has at least one of the following features:

(i) an absorption capacity of 0.9% saline solution greater than 6 g/g, preferably greater than 15 g/g,
(ii) an absorption capacity of 0.9% saline solution under 115 psi load greater than 60, preferably greater than 113,
(iii) an absorption rate of 0.9% saline solution greater than 1, preferably greater than 10.

The above features have been determined by the method according with EDANA NWSP 070.7-8-9R0/1(15).

The superabsorbent fibers are made of cross-linked polymers or copolymers of carboxylic acid salts, preferably sodium polyacrylate.

The superabsorbent fibers may be the one marketed by Technical Absorbents Ltd (UK) under the trade name SAF®.

The polyester fibers are preferably polyethylene terephthalate fibers.

The polyolefin fibers are preferably polypropylene fibers.

The fiber blend comprising the washable absorbent pad comprises superabsorbent fibers between 10 and 90 % by total fibers weight, and polyester and/or polyolefin fibers between 10 and 90 % by total fiber weight. Preferably, the blend of fibers comprises superabsorbent fibers between 45 and 55 % by total fiber weight, and polyester and/or polyolefin fibers between 45 and 55 % by total fiber weight.

In a preferred embodiment, the blend of fibers comprising the washable absorbent pad comprises superabsorbent fibers in an amount equal to about 52 % by total fiber weight, and polypropylene and polyethylene terephthalate fibers in an amount equal to about 48 % by total fiber weight.

The decontaminating and/or functionalizing materials - if present - are contained in the washable absorbent pad in an amount between 0 and 20% by total pad weight.

The absorbent materials - if present - are superabsorbent polymers in powder form. The superabsorbent polymers in powder form can be present in an amount comprised between 10 and 30 % by total pad weight, preferably between 10 and 15 % by total pad weight.

The two external closure sheets enclosing the washable absorbent pad are an acquisition and diffusion film and a barrier film, respectively, wherein the acquisition and diffusion film is positioned adjacent to the top layer 30 and the barrier film is adjacent to the back layer 12.

The acquisition and diffusion film may be made of a polypropylene, polyethylene and polyester fiber laminate. Preferably, the acquisition and diffusion film may be made of polypropylene and polyester fiber laminate.

The barrier film, which must be waterproof and soft to the touch and may also be breathable, may consist of a laminate of polyethylene and polyester fibers (preferably a laminate of polypropylene and polyethylene terephthalate fibers), a polyurethane laminate, or a low-weight low-density polyethylene film/elastane.

The barrier film may also consist of a continuous glue polyolefines or polyammide types film directly applied to the washable absorbent pad during the manufacturing process.

The washable absorbent pad has a weight comprised between 100 and 700 $g/m^2$.

The washable absorbent core 28 has a total weight comprised between 150 and 750 $g/m^2$ and a total thickness comprised between 1,5 and 8,5 mm.

The washable absorbent garment according to the present invention can be manufactured by the known techniques used for manufacturing textiles articles, typically by manually cutting and sewing fabric materials, or by the manufacturing process disclosed in the European patent application n. EP21207932.1 in the name of the same applicant.

**Claims**

1. A washable absorbent garment (10) comprising:

   - a back layer (12) of washable fabric forming a pant structure and having rear and front waist sections (14, 16) and a crotch section (18) intermediate between the rear and front waist sections (14, 16), wherein the rear and front waist sections (14, 16) have respective side edges (24, 26) joined to each other,
   - a washable absorbent core (28) including a washable absorbent pad, and
   - a top layer (30) of washable fabric which covers the washable absorbent core (28),
   wherein the top layer (30) and the washable absorbent core (28) are permanently fixed in the crotch section (18) of the back layer (12),
   wherein the washable absorbent pad comprises a blend of superabsorbent fibers, and polyolefin and/or polyester fibers, and
   wherein the washable absorbent pad has a performance ratio, defined as ratio between absorbent capacity (g/g) and thickness (mm), comprised between 1 and 15 g/g·mm, wherein the thickness is equal to or lower than 7 mm.

2. The washable absorbent garment of claim 1, wherein the fibers blend comprising the washable absorbent pad has at least one of the following features:

   (i) an absorption capacity of 0.9% saline solution greater than 6 g/g,
   (ii) an absorption capacity of 0.9% saline solution under 115 psi load greater than 60,
   (iii) an absorption rate of 0.9% saline solution greater than 1.

3. The washable absorbent garment of any of the preceding claims, wherein the fiber blend comprises superabsorbent fibers between 10 and 90 % by total fibers weight, and polyester and/or polyolefin fibers between 10 and 90 % by total fibers weight.

4. The washable absorbent garment of any of the preceding claims, wherein the fiber blend comprises superabsorbent fibers between 45 and 55 % by total fibers weight, and polyester and/or polyolefin fibers between 45 and 55 % by total fibers weight.

5. The washable absorbent garment of any of the preceding claims, wherein the fiber blend comprises superabsorbent fibers in an amount equal to about 52 % by total fibers weight, and polypropylene and polyethylene terephthalate fibers in an amount equal to about 48 % by total fibers weight.

6. The washable absorbent garment of any of the preceding claims, wherein the washable absorbent pad further comprises superabsorbent polymer(s) in powder form.

7. The washable absorbent garment of claim 6, wherein the superabsorbent polymer(s) in powder form is(are) present in an amount comprised between 10 and 30 % by total pad weight.

8. The washable absorbent garment of any of the preceding claims, wherein the washable absorbent pad has a weight comprised between 100 and 700 $g/m^2$.

9. The washable absorbent garment of any of the preceding claims, wherein the washable absorbent pad is sandwiched between an acquisition and diffusion film and a barrier film.

10. The washable absorbent garment of any of the preceding claims, wherein the washable absorbent core (28) has a total weight comprised between 150 and 750 $g/m^2$.

11. The washable absorbent garment of any of the preceding claims, wherein the washable absorbent core (28) has a total thickness comprised between 1,5 and 8,5 mm.

fig. 1

fig.2

fig.3

Europäisches
Patentamt

European
Patent Office

Office européen
des brevets

# EUROPEAN SEARCH REPORT

Application Number

EP 21 21 2015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/290447 A1 (SEPELLO CASSANDRA A [US] ET AL) 23 September 2021 (2021-09-23) | 1,2,9,11 | INV. A61F13/49 A61F13/496 |
| Y | * paragraphs [0035], [0041], [0043], [0071], [0078], [0079]; claims 1,2, 8,11,13,15, 20; figures 2A, 2B, 4; examples * | 3-8,10 | |
| Y | US 2014/018757 A1 (DE BRUIN PAULA KATHLEEN [US] ET AL) 16 January 2014 (2014-01-16) * paragraphs [0013], [0059], [0188], [0198], [0211]; claims 1,11-13, 17; figure 1 * | 8,10 | |
| Y | WO 2019/126226 A1 (LYV LIFE INC [US]) 27 June 2019 (2019-06-27) * paragraphs [0021], [0032], [0034] * | 3-7 | |
| A | CA 2 827 795 A1 (PROD DRC INC [CA]) 25 November 2013 (2013-11-25) * figures 2A, 3, 4 * | 1-11 | |
| A,D | GB 2 176 692 A (GANMILL LTD) 7 January 1987 (1987-01-07) * the whole document * | 1-11 | TECHNICAL FIELDS SEARCHED (IPC) A61F |
| A | WO 2008/008743 A1 (DEERIN ROBERT F [US]) 17 January 2008 (2008-01-17) * claims 1,3,4,15,22; figure 1 * | 1-11 | |

The present search report has been drawn up for all claims

| Place of search | Date of completion of the search | Examiner |
|---|---|---|
| Munich | 25 May 2022 | Adechy, Miriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 2015

25−05−2022

| Patent document cited in search report | | Publication date | Patent family member(s) | | Publication date |
|---|---|---|---|---|---|
| US 2021290447 | A1 | 23−09−2021 | US 2021290447 | A1 | 23−09−2021 |
| | | | WO 2021155397 | A1 | 05−08−2021 |
| US 2014018757 | A1 | 16−01−2014 | AU 2013288292 | A1 | 05−02−2015 |
| | | | BR 112015000595 | A2 | 27−06−2017 |
| | | | CN 104619299 | A | 13−05−2015 |
| | | | EP 2872095 | A2 | 20−05−2015 |
| | | | KR 20150048111 | A | 06−05−2015 |
| | | | MX 339286 | B | 18−05−2016 |
| | | | RU 2586055 | C1 | 10−06−2016 |
| | | | US 2014018757 | A1 | 16−01−2014 |
| | | | WO 2014009899 | A2 | 16−01−2014 |
| WO 2019126226 | A1 | 27−06−2019 | CA 3086035 | A1 | 27−06−2019 |
| | | | US 2020375811 | A1 | 03−12−2020 |
| | | | WO 2019126226 | A1 | 27−06−2019 |
| CA 2827795 | A1 | 25−11−2013 | NONE | | |
| GB 2176692 | A | 07−01−1987 | NONE | | |
| WO 2008008743 | A1 | 17−01−2008 | CN 101522148 | A | 02−09−2009 |
| | | | EP 2043580 | A1 | 08−04−2009 |
| | | | EP 2433600 | A1 | 28−03−2012 |
| | | | ES 2526205 | T3 | 08−01−2015 |
| | | | HK 1135593 | A1 | 11−06−2010 |
| | | | PT 2043580 | E | 14−01−2015 |
| | | | US 2008015538 | A1 | 17−01−2008 |
| | | | US 2009299311 | A1 | 03−12−2009 |
| | | | US 2012123380 | A1 | 17−05−2012 |
| | | | WO 2008008743 | A1 | 17−01−2008 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2176692 A
- EP 21207932